# EUROPEAN PATENT APPLICATION

(11) **EP 4 752 898 A1**
(43) Date of publication of application: **03.06.2026**
(21) Application number: 25219578.9
(22) Date of filing: 28.11.2025
(51) Int. Cl.: G16H 20/40, A61B 18/14

(54) **PULSED FIELD ABLATION INDEX (PI) METRIC FOR LESION ASSESSMENT**

(30) Priority: 02.12.2024 US 202463727047 P
(71) Applicant: St. Jude Medical, Cardiology Division, Inc., St. Paul, MN 55117 (US)
(72) Inventor: WOODS, Virginia, Grant, 55110 (US); FISH, Jeffrey M., Maple Grove, 55311 (US); TRANTER, John, Minneapolis, 55417 (US); VOTH, Eric J., Maplewood, 55109 (US); MARASS, Timothy S., Minneapolis, 55419 (US); FARAHMAND, Sina, Woodbury, 55129 (US); HARELAND, Scott, Lino Lakes, 55014 (US)
(74) Representative: Mathys & Squire

(57) **Abstract**

A method of assessing lesion formation associated with pulsed field ablation (PFA) therapy delivered to a selected location by a PFA catheter includes measuring pre-therapy impedance using one or more electrodes located on the catheter and delivering a first PFA application. An intra-therapy/post-therapy impedance is measured using the one or more electrodes located on the catheter and a PFA index (PI) metric is calculated based on a model that combines the measured pre-therapy impedance, the measured intra-therapy/post-therapy impedance, and attributes of the first PFA application delivered, wherein the PI metric is representative of a likelihood that the lesion has reached a selected size.

## Description

### TECHNICAL FIELD

The subject matter disclosed herein relates to electrophysiology procedures and, in particular, to assessing lesion formation associated with pulsed field ablation (PFA) therapy.

### BACKGROUND

Ablation therapy may be used to treat various conditions afflicting the human anatomy. One such condition in which ablation therapy may be used is the treatment of cardiac arrhythmias. When tissue is ablated, or at least subjected to ablative energy generated by an ablation generator and delivered by an ablation catheter, lesions form in the tissue. Electrodes mounted on or in ablation catheters are used to create tissue necrosis in cardiac tissue to correct conditions such as atrial arrhythmia (including, but not limited to, ectopic atrial tachycardia, atrial fibrillation, and atrial flutter). Arrhythmias can create a variety of dangerous conditions including loss of synchronous atrioventricular contractions and stasis of blood flow. It is believed that the primary cause of atrial arrhythmias is stray electrical signals within the left or right atrium of the heart. The ablation catheter imparts ablative energy (e.g., radiofrequency energy, cryoablation, lasers, chemicals, high-intensity focused ultrasound, etc.) to cardiac tissue to create a lesion in the cardiac tissue. This lesion disrupts undesirable electrical pathways and thereby limits or prevents stray electrical signals that may lead to arrhythmias.

Electroporation is a non-thermal ablation technique that involves applying strong electric fields that induce pore formation in the cellular membrane. The electric field may be induced by applying a relatively short duration pulse which may last, for example, from a nanosecond to several milliseconds. Such a pulse may be repeated to form a pulse train (or "burst"). When such an electric field is applied to tissue in an in vivo setting, the cells in the tissue are subjected to a trans-membrane potential, which opens the pores on the cell wall. Electroporation may be reversible (i.e., the temporarily-opened pores will reseal) or irreversible (i.e., the pores will remain open, causing cellular destruction). In certain therapeutic applications, a suitably configured pulse train alone may be used to cause cell destruction, for instance by causing irreversible electroporation (IRE). In the context of cardiac ablation to create transmural lesions, this is known as pulsed field ablation (PFA).

To deliver a PFA treatment the physician places the catheter (in particular, the electrode(s) selected to deliver the PFA waveform) adjacent to the tissue to be treated and initiates delivery of the PFA waveform. It would be beneficial to provide a metric that describes to the physician the likelihood that a lesion of a desired size has been created at a particular location.

### SUMMARY

In some aspects, the techniques described herein relate to a computer-implemented method of assessing lesion formation associated with pulsed field ablation (PFA) therapy delivered to a selected location by a PFA catheter, the method including: measuring pre-therapy impedance using one or more electrodes located on the catheter; measuring intra-therapy/post-therapy impedance using the one or more electrodes located on the catheter; and calculating a PFA index (PI) metric based on a model that combines the measured pre-therapy impedance, the measured intra-therapy/post-therapy impedance, and attributes of a delivered PFA therapy, wherein the PI metric is representative of a likelihood that the lesion has reached a selected size.

In some aspects, the techniques described herein relate to a method, wherein the measured pre-therapy impedance and measured intra-therapy/post-therapy impedance is representative of tissue impedance associated with tissue receiving the delivered PFA therapy.

In some aspects, the techniques described herein relate to a method, wherein the model utilizes a change in tissue impedance calculated on a minimum tissue impedance measured and a maximum tissue impedance measured.

In some aspects, the techniques described herein relate to a method, further including: measuring one or more other inputs associated with the delivered PFA therapy, wherein the model further combines the one or more other inputs in generating the PI metric.

In some aspects, the techniques described herein relate to a method, wherein the one or more other inputs includes contact force measured between the catheter and adjacent tissue.

In some aspects, the techniques described herein relate to a method, wherein contact force is measured as a percentage of time the measured contact force is greater than or equal to a threshold value.

In some aspects, the techniques described herein relate to a method, wherein the PI metric calculated by the model is filtered to only permit increases in value of the PI metric.

In some aspects, the techniques described herein relate to a method, wherein the delivered PFA therapy includes a first PFA application included of a first plurality of PFA bursts, wherein the pre-therapy impedance and intra-therapy impedances are measured during a pre-burst interval preceding each of the first plurality of PFA bursts, wherein a post-burst impedance is measured after a final PFA burst delivered as part of the first PFA application.

In some aspects, the techniques described herein relate to a method, wherein attributes of the first PFA application delivered utilized by the model includes a number of PFA bursts delivered.

In some aspects, the techniques described herein relate to a method, further including: measuring one or more additional inputs during the pre-burst intervals preceding each of the first plurality of PFA bursts, wherein the one or more additional inputs includes contact force.

In some aspects, the techniques described herein relate to a method, further includes: delivering as second PFA application to the selected location if the PI metric is less than a threshold value, wherein the second PFA application includes a second plurality of PFA bursts.

In some aspects, the techniques described herein relate to a method, further including: measuring first contact force associated with the catheter during delivery of the first PFA application delivered; measuring second contact force associated with the catheter during delivery of the second PFA application delivered, wherein calculating the PI metric following delivery of the second PFA application utilizes a cumulative contact force based on the first contact force and the second contact force.

In some aspects, the techniques described herein relate to a system for assessing lesion formation associated with pulsed field ablation (PFA) therapy delivered to a selected location by a PFA catheter, the system including: a lesion assessment module running on a computer system, the lesion assessment module configured to: receive pre-therapy impedances measured using one or more electrodes located on the catheter; receive intra-therapy/post-therapy impedances measured using the one or more electrodes located on the catheter; and calculate a PFA index (PI) metric based on a model that combines the measured pre-therapy impedance, the measured intra-therapy/post-therapy impedance, and attributes of the delivered PFA therapy, wherein the PI metric is representative of a likelihood that the lesion has reached a given size.

In some aspects, the techniques described herein relate to a system, wherein the measured pre-therapy impedance and measured intra-therapy/post-therapy impedance is representative of tissue impedance associated with tissue receiving the delivered PFA therapy.

In some aspects, the techniques described herein relate to a system, wherein the model utilizes a change in tissue impedance calculated on a minimum tissue impedance measured and a maximum tissue impedance measured.

In some aspects, the techniques described herein relate to a system, wherein the lesion assessment module is further configured to: receive one or more other inputs measured with respect to the delivered PFA therapy, wherein the model further combines the one or more other inputs in generating the PI metric.

In some aspects, the techniques described herein relate to a system, wherein the one or more other inputs measured includes contact force measured between the catheter and adjacent tissue.

In some aspects, the techniques described herein relate to a system, wherein contact force is measured as a percentage of time the measured contact force is greater than or equal to a threshold value.

In some aspects, the techniques described herein relate to a system, wherein the PI metric calculated by the model is filtered to only permit increases in value of the PI metric.

In some aspects, the techniques described herein relate to a system, wherein the delivered PFA therapy includes a first PFA application included of a first plurality of PFA bursts, wherein the pre-therapy impedance and intra-therapy impedances are measured during a pre-burst interval preceding each of the first plurality of PFA bursts, wherein a post-burst impedance is measured after a final PFA burst delivered as part of the first PFA application.

In some aspects, the techniques described herein relate to a system, wherein attributes of the first PFA application delivered utilized by the model includes a number of PFA bursts delivered.

In some aspects, the techniques described herein relate to a system, wherein the lesion assessment module is further configured to: receive one or more additional inputs measured during the pre-burst intervals preceding each of the first plurality of PFA bursts, wherein the one or more additional inputs includes contact force.

In some aspects, the techniques described herein relate to a method, wherein the lesion assessment module is further configured to: receive a first contact force associated with the catheter during delivery of a first PFA application delivered; receive a second contact force associated with the catheter during delivery of a second PFA application, wherein calculating the PI metric following delivery of the second PFA application utilizes a cumulative contact force based on the first contact force and the second contact force.

In some aspects, the techniques described herein relate to a computer implemented method of assessing lesion formation associated with pulsed field ablation (PFA) therapy delivered to a selected location by an electrode located on a catheter, the method including: measuring tissue impedance both prior to and after delivery of a first PFA application to determine a change in tissue impedance; measuring contact force between the catheter and adjacent tissue prior to delivery of the first PFA application; and calculating a PFA index (PI) metric based on a model that combines the change in tissue impedance, the contact force, and one or more attributes of the first PFA application, wherein the PI metric is representative of a likelihood that the lesion has reached a selected size (i.e., selected depth, selected width, or a combination of selected depth and selected width).

In some aspects, the techniques described herein relate to a computer implemented method, further including delivering a first PFA application to the selected location.

In some aspects, the techniques described herein relate to a computer implemented method, wherein attributes of the first PFA application delivered utilized by the model includes a number of PFA bursts delivered, and wherein the system is configured to deliver a second PFA application to the selected location if the PI metric is less than a threshold value, wherein the second PFA application includes a second plurality of PFA bursts.

In some aspects, the techniques described herein relate to a method, wherein the model is a probabilistic model that maps the measured change in tissue impedance, the measured contact force, and the one or more attributes of the first PFA application to a probability the lesion has reached the selected size.

In some aspects, the techniques described herein relate to a method, further including: receiving an input from a user regarding the selected lesion size; and modifying the model to generate a PI metric representative of a likelihood that the lesion has reached the selected size.

In some aspects, the techniques described herein relate to a method, wherein the first PFA application is included of a plurality of bursts.

In some aspects, the techniques described herein relate to a method, wherein the tissue impedance is measured before each burst and after a final burst of the first PFA application and the contact force is measured before each burst.

In some aspects, the techniques described herein relate to a method, wherein change in tissue impedance is calculated by identifying a minimum tissue impedance and a maximum tissue impedance measured and subtracting the minimum tissue impedance from the maximum tissue impedance.

In some aspects, the techniques described herein relate to a method, wherein the contact force is calculated as a percentage of time across all measurements that the contact force is above a threshold.

In some aspects, the techniques described herein relate to a method, wherein a second PFA application is delivered if the PI metric is less than a threshold value.

In some aspects, the techniques described herein relate to a method of assessing lesion formation associated with pulsed field ablation (PFA) therapy delivered to a selected location by a PFA catheter, the method including: measuring pre-therapy impedance using one or more electrodes located on the catheter; delivering a first PFA application; measuring intra-therapy/post-therapy impedance using the one or more electrodes located on the catheter; and calculating a PFA index (PI) metric based on a model that combines the measured pre-therapy impedance, the measured intra-therapy/post-therapy impedance, and attributes of the first PFA application delivered, wherein the PI metric is representative of a likelihood that the lesion has reached a selected size.

In some aspects, the techniques described herein relate to a system for assessing lesion formation associated with pulsed field ablation (PFA) therapy delivered to a selected location by an electrode located on a catheter, the system including a lesion assessment module running on a computer system, the lesion assessment module configured to: measure tissue impedance both prior to and after delivery of a first PFA application to determine a change in tissue impedance; measure contact force between the catheter and adjacent tissue prior to delivery of the first PFA application; and calculate a PFA index (PI) metric based on a model that combines the change in tissue impedance, the contact force, and one or more attributes of the first PFA application, wherein the PI metric is representative of a likelihood that the lesion has reached a selected size.

In some aspects, the techniques described herein relate to a method of assessing lesion formation associated with pulsed field ablation (PFA) therapy delivered to a selected location by an electrode located on a catheter, the method including: delivering a first PFA application to the selected location, the first PFA application defined by one or more attributes; measuring tissue impedance both prior to and after delivery of the first PFA application to determine a change in tissue impedance; measuring contact force between the catheter and adjacent tissue prior to delivery of the first PFA application; and calculating a PFA index (PI) metric based on a model that combines the change in tissue impedance, the contact force, and the one or more attributes of the first PFA application, wherein the PI metric is representative of a likelihood that the lesion has reached a selected size.

### BRIEF DESCRIPTION OF THE DRAWINGS

**Fig. 1A** is a diagrammatic and block diagram view of a system for delivering pulsed field ablation (PFA) therapy.
**Fig. 1B** is a schematic representation of an ablation catheter for use in connection with the system for delivering PFA therapy shown in Figure 1A.
**FIG. 2** is a side view of lesion formation.
**FIG. 3** is a block diagram illustrating inputs provided to the PFA index (PI) model to calculate the PI metric.
**FIG. 4** is a graph illustrating the bounding of the PI metric.
**FIG. 5** is a flowchart illustrating calculation of the PI metric based on received impedance measurements.
**FIG. 6** is a flowchart illustrating calculation of the PI metric based on impedance measurements, contact force measurements, and attributes of the delivered PFA therapy.
**FIG. 7** is a schematic illustrating delivery of a plurality of PFA bursts and measurement windows preceding each burst and subsequent to the last burst.
**FIG. 8** is a schematic illustrating the delivery of first and second applications of PFA therapy, each application comprised of a plurality of bursts.
**FIG. 9** is a flowchart illustrating the aggregation of inputs measured over two or more PFA applications based on timing/location thresholds.
**FIG. 10** is a schematic illustrating timing thresholds between delivery of first and second applications of a PFA therapy.

### DETAILED DESCRIPTION

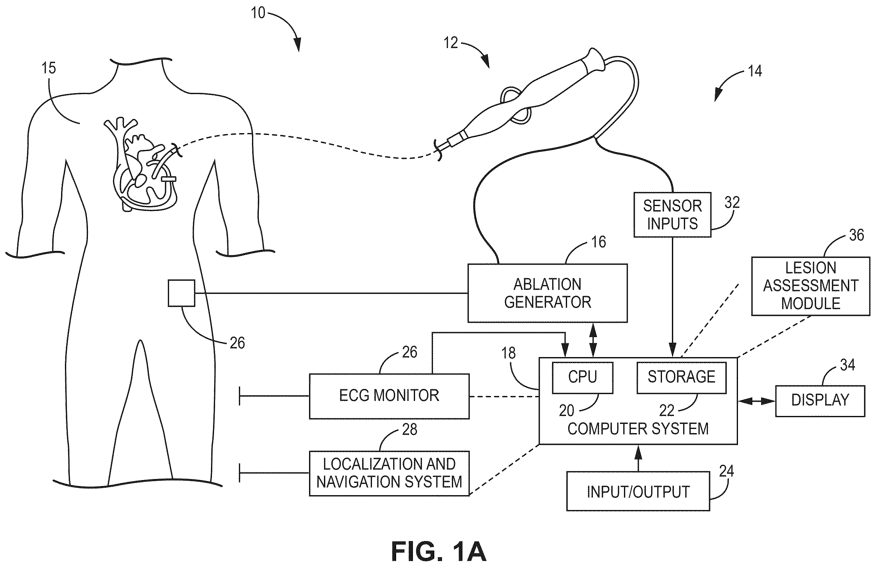

**Fig. 1A** is a diagrammatic and block diagram view of a system 10 for delivering pulsed field ablation (PFA) therapy. In general, the system 10 includes an ablation catheter 12, such as the TactiFlex^{™} Ablation Catheter, Sensor Enabled^{™} (Abbott Laboratories; Abbott Park, Illinois) having a proximal end and a distal end navigated to a location within a patient 15. The distal end includes, among other things, one or more electrodes configured to deliver pulsed field ablation therapy (PFA) and one or more sensors. In addition, the system 10 includes a plurality of electronics 14, including ablation generator 16, computer system 18, which includes a processor (CPU) 20 and memory/storage 22, input/output systems 24, and a display 34. The ablation generator 16 is configured to receive commands from the computer system 18 and to deliver, via catheter 12, PFA applications to one or more electrodes located at the distal end of catheter 12. A PFA application may include the generation of various types of PFA waveforms (e.g., plurality of bursts, single pulse, etc.) which are well understood and not discussed in detail here. Computer system 18 may provide instructions to ablation generator 16 regarding the type of PFA waveform to generate and ablation generator 16 may provide feedback to computer system 18 regarding delivered PFA waveforms. The delivery of PFA applications to one or more electrodes located at the distal end of catheter 12 creates lesions in the tissue adjacent to the electrodes.

As shown in **FIG. 1A****,** computer system 18 may comprise, for example, a conventional general-purpose computer, a special-purpose computer, a distributed computer, or any other type of computer. Computer system 18 may include one or more processors 20, such as a single central processing unit ("CPU"), or a plurality of processing units, commonly referred to as a parallel processing environment, which may execute instructions to practice the various aspects described herein. The one or more processors 20 are configured to execute instructions stored on storage medium 22 to implement functionality described with respect to various elements, including lesion assessment module 36 and localization and navigation system 28. In addition, computer system 18 received inputs from various sources including for example ECG monitor 26, ablation generator 16, input/output 24, display 34, sensors inputs 32, etc.

As described in more detail below, computer system 18 implements a lesion assessment module 36 that assesses the lesions formed in the tissue by the delivered PFA applications. In one example, the lesion assessment module 36 generates a PFA Index (PI) metric that represents the probability that a delivered PFA application resulted in a lesion having a desired size, including (i) a desired depth, (ii) a desired width, or (iii) a combination of both depth and width. The PI metric may be utilized as a feedback signal to determine whether a particular PFA application created a desired lesion or whether additional PFA applications should be applied. The PI metric may also be displayed to the physician/user via display 34, via a graphical user interface (GUI) that illustrates the location of the lesion and the PI metric associated with a given lesion. For example, the GUI may utilize color coding to illustrate to the physician whether a given lesion likely satisfies the lesion size requirements of a given application.

As described in more detail below, the lesion assessment module 36 generates the PI metric using a model that combines a plurality of inputs. In one example, the model is a multivariable probabilistic model that predicts lesion formation based on the plurality of inputs. At least some of the inputs are measured by one or more sensors located on the catheter, as indicated by sensor inputs 32 shown in **FIG. 1A****.** Exemplary inputs include impedance measurements, impedance change measurements, tissue impedance measurements, tissue impedance change measurements, current measurements, contact force measurements, contact force stability measurements, irrigation flow rate, delivery path (bipolar versus monopolar), and temperature measurements. For example, catheter 12 may include one or more electrodes located at a distal end of the catheter 12 for making impedance measurements, typically by providing an AC signal to one or more electrodes and measuring the voltage at the one or more electrodes. The voltage may be measured between two or more electrodes located at the catheter or with respect to another electrode either located on another catheter (i.e., reference catheter) or a reference electrode affixed to the skin of the patient. Additionally, the model may utilize additional inputs related to the PFA application or applications delivered to a given location, including for example deceleration zones (DZ), pulse width, pulse period, loop period, pulses per burst, inter-burst timing, number of bursts delivered, voltage of delivered bursts, electric field, energy-per-pulse, energy-per-burst, polarity, etc. Based on these inputs, the model generates a PI metric that represents probability that the PFA application or applications delivered created a lesion of a desired size (e.g., the created lesion satisfies (i) a desired depth, (ii) a desired width, or (iii) a combination of both depth and width.

In addition, the plurality of electronics 14 may also include components such as an electrocardiogram (ECG) monitor 26 and localization and navigation systems 28. The localization and navigation system 28 may utilize one or more well-known localization systems, including for example impedance-based localization and magnetic-based localization. Impedance-based localization utilizes a plurality of pairs of surface patch electrodes to generate a voltage field throughout the patient's body that may be sensed by electrodes located at the distal end of the catheter and utilized to determine the 3D location of the electrodes within the patient's body 115. Magnetic-based localization utilizes magnetic sensors (e.g., coils) located at the distal end of the catheter to sense an externally generated magnetic field to determine the 3D location of the magnetic sensors within the patient's body 115.

**FIG. lB** schematically illustrates ablation catheter 12. Ablation catheter 12 includes one or more electrodes 112, 114, and 116, which may be used for a variety of diagnostic and/or therapeutic purposes including, without limitation, electrophysiological mapping, impedance-based localization, impedance measurements, and delivery of pulsed field ablation (PFA) therapies. For example, and in some embodiments of the disclosure, two or more of the electrodes 112, 114, and 116 may be configured as a bipolar electrode assembly for use in bipolar-based PFA therapy. Specifically, electrodes 112, 114 or 116 may be individually electrically coupled to ablation generator 16 (e.g., via suitable electrical wire 42 or other suitable electrical conductors connected through electrical connector 40 as discussed in further detail below) and configured to be selectively energized (e.g., by ablation generator 16 under control of closed-loop ablation processor 20) with opposite polarities to generate a potential and corresponding electric field therebetween for PFA therapy. That is, one of electrodes 112, 114 or electrodes 114, 116 can be configured to function as a cathode, and the other can be configured to function as an anode for a given PFA burst.

Electrodes 112, 114, and 116 may be any suitable electrodes. In an exemplary embodiment, electrodes 112, 114 are ring electrodes, though electrodes 112, 114 may have any other shape or configuration without departing from the scope of the present disclosure. Moreover, although each of electrode 112 and electrode 114 are illustrated as single electrodes, either or both of electrode 112 and electrode 114 may be alternatively embodied as two or more discrete electrodes. Distal electrode 116 may be a tip electrode.

Those of ordinary skill in the art will recognize that variations in the shape, size, and/or configuration of electrodes 112, 114, and 116 may result in variations in parameters of the applied ablation therapy. For example, increasing the surface area of the electrodes 112, 114, and 116 would decrease impedance, in turn decreasing the current that would need to be applied to achieve the voltage level required to cause tissue destruction in PFA therapy. In addition, PFA therapy delivered via the one or more electrodes 112, 114, and 116 may be bipolar or unipolar. For bipolar delivery, a pair of electrodes (e.g., electrode pair 112 and 114, or electrode pair 114 and 116) are utilized to deliver the bipolar PFA therapy. For unipolar delivery, a single electrode (e.g., electrode 112, 114, or 116) is utilized to deliver the unipolar PFA therapy.

In addition to delivering PFA therapy, one or more of the electrodes 112, 114, 116 may be utilized as sensors to collect information utilized to assess the delivery and effect of a delivered PFA therapy. For example, one or more of the electrodes 112, 114, and 116 may be utilized to measure impedances associated with the one or more electrodes. For example, in one embodiment a current signal is delivered between electrodes 114 and 116 and corresponding voltages are measured with respect to each electrode (e.g., voltage measured between each electrode and a reference electrode) to determine an impedance between the respective electrodes 114, 116 (i.e., a three-terminal measurement). In other examples a current signal is provided to one of the electrodes (e.g., electrode 116) and a voltage response is measured between the electrode and a reference electrode (e.g. surface patch electrode) to determine an impedance associated with the region adjacent to the selected electrode (i.e., a two-terminal measurement). The impedance measurements may be utilized to determine proximity of the electrode to adjacent tissue, contact status of the electrode with adjacent tissue, and/or tissue impedance. For example, if impedance is measured between electrodes 116 and 114 and electrode 116 is in contact with the adjacent tissue, the measured impedance will reflect the tissue impedance value.

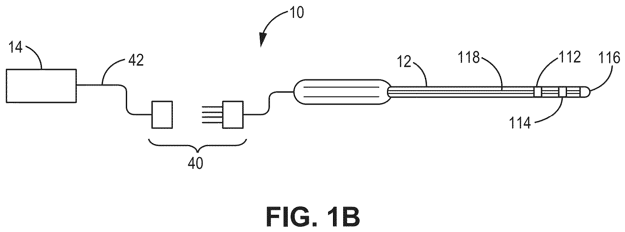

The catheter 12 may include a number of other sensors, including force contact sensors, shape sensor, temperature sensors, etc. For example, in the embodiment shown in **FIG. 1B** the catheter 12 includes a sensor 118 for detecting contact force between the catheter 12 and adjacent tissue. The sensor 118 may be implemented in a variety of ways, including utilization of a fiber optic cable including, for example, a plurality of fiber Bragg gratings (FBGs) configured to detect force contact between the catheter 12 and adjacent tissue and/or shape of the catheter 12. In other embodiments, mechanical sensors may be utilized, such as piezo-electric sensors and/or spring-based sensors to detect force contact between the catheter 12 and adjacent tissue.

Also shown in **Fig. lB** is an electrical connector 40. As shown, the plug side of electrical connector 40 is connected to catheter 12, while the receptacle side of electrical connector 40 is connected to electronics 14 via cable 42. This arrangement could be reversed without departing from the scope of the instant disclosure. The ordinarily skilled artisan will appreciate that, when the plug and receptacle portions of electrical connector 40 are mated, catheter 12 becomes electrically coupled to electronics 14, enabling power, data, and other electrical signals to pass between the two.

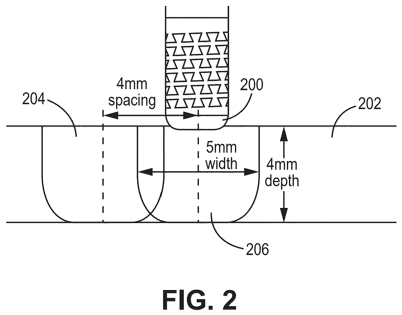

**FIG. 2** is a side view of lesions 204, 206 formed in tissue 202. In the embodiment shown in FIG. 2, electrode 200 located at a distal end of a catheter delivers the PFA therapy. Lesion size is a function of PFA therapy delivered (e.g., type, duration, number of bursts, etc.) and therefore attributes of the PFA therapy may be utilized as an input to the PI model. In addition, lesion size is a function of the position of the electrode 200 with respect to the tissue 202 during delivery of the PFA therapy. The PI model may therefore utilize sensor inputs related to the position of the electrode 200 with respect to the tissue 202 during delivery of the PFA therapy. For example, sensor inputs related to contact force, tissue proximity, or tissue contact between electrode 200 and the tissue 202 during delivery of PFA therapy may be utilized as inputs to the PI model to generate the PI metric.

In addition, the lesions result in changes to the tissue that may be sensed and utilized as an input to the PI model. For example, the conductivity/impedance of the tissue changes in response to lesion formation. Sensor inputs related to measuring tissue impedance, changes in tissue impedance, etc. may be utilized as inputs to the PI model to generate the PI metric. The PI model combines inputs related to the PFA therapy delivered, the position of the electrode 200 during delivery of the PFA therapy, and measured attributes of the tissue itself (e.g., tissue impedance) to generate the PI metric, which represents a probability that a created lesion satisfied lesion size requirements.

In this example, the tissue 202 has a depth of approximately 4mm and therefore it is desirable that the lesion formed from the PFA therapy reaches a depth of at least 4mm. The model utilized to generate the PI metric for such an application is therefore configured to calculate a probability that a lesion PFA application or applications has reached a depth of 4mm or greater. In addition to lesion depth, the model may also be utilized to calculate the probability that a lesion formed from the PFA therapy reaches a desired width (e.g., 5mm). In the example shown in **FIG. 2****,** a first PFA application creates first lesion 204. The electrode is moved 4mm and a second PFA application is utilized to form a second lesion 206. Providing a PI metric that provides a probability that each lesion has reached a width (e.g., 5mm) greater than the distance between adjacent lesions (e.g., 4mm) ensures that the first lesion 204 and second lesion 206 overlap with one another to ensure contiguous lesions. In some examples, rather than a width threshold (e.g., 5mm), a width range may be provided that allows the physician to tune depth/width trade-offs in predicted lesion dimensions to align with the physician's workflow (e.g., PI metric satisfying the lower end of the width range may be acceptable for a lesion located close to an adjacent lesion, etc.). In some examples, rather than calculate the probability that a lesion has reached a selected depth or a selected depth, the model calculates a probability that a lesion has reached a combination of both the selected depth and the selected width. As described above, the model may be utilized to calculate a probability that a lesion formed from the PFA therapy satisfies (i) a desired depth, (ii) a desired width, or (iii) a combination of both depth and width.

The PI metric provides the physician with a tool to assess lesion formation. In particular, the PI metric is not based only on the PFA therapy delivered, but on additional inputs that are utilized to determine the probability that created lesions satisfy the desired size requirements of a given application.

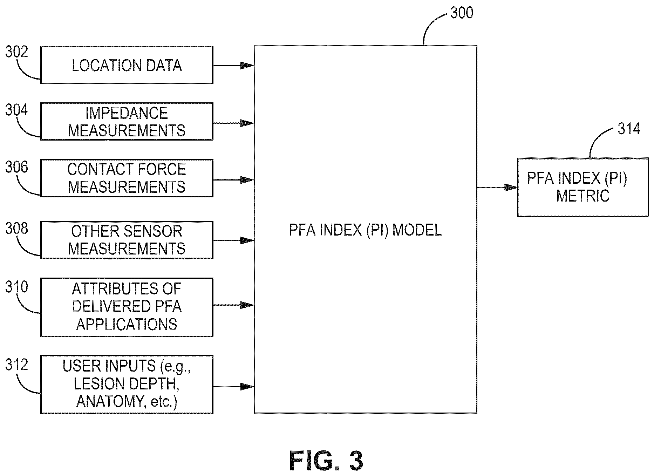

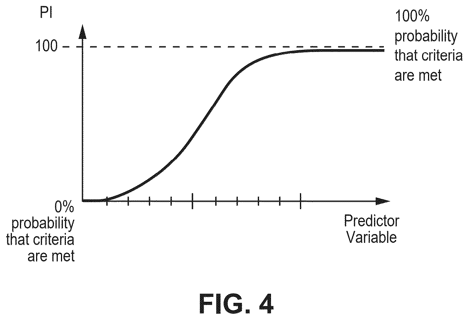

**FIG. 3** is a block diagram illustrating inputs provided to the PFA index (PI) model 300 to calculate the PI metric. A non-limiting list of inputs to the PI model 300 includes location data 302, impedance measurements 304, contact/contact force measurements 306, other sensor measurements 308, attributes of delivered PFA applications 310, and user inputs 312 (e.g., desired lesion depth, desired lesion width, anatomy, etc.). PI model 300 generates a PI metric by combining two or more of the plurality of inputs. As described above, in some embodiments the PI model 300 is a probabilistic model that provides as an output a probability that a set of conditions (e.g., lesion depth, lesion width, or a combination of lesion depth and lesion width) have been satisfied. For example, **FIG. 4** is a graph illustrating the increase in the PI metric (y-axis) in response to increases in some predictor variable or combination of predictor variables (x-axis). The probability estimate corresponds with zero initially and increases to almost 100% as the predictor variable increases in value. Alternatives to a PI index represented as a probability include the PI index being represented as an expected lesion size (e.g., expected lesion depth, expected lesion width, or combination of lesion depth and lesion width).

Returning to **FIG. 3****,** each of the possible inputs to the PI model 300 is described in turn. The location data 302 includes input related to the location of the electrode utilized to deliver a PFA therapy. Location data 302 may include the position of the electrode relative to some anatomy utilized to deliver a PFA therapy. The location data may be utilized to generate an output displaying to the user the location of a delivered PFA therapy as well as the PI metric associated with the location. A physician may utilize the display to select locations that should receive additional PFA therapy. In addition, the location data may be utilized by the model to determine whether successive PFA applications should be cumulative or associated with separate locations. For example, if a first PFA application is delivered to a first location, and a determination is made based on the PI metric that the first lesion is not sufficient, a second PFA application will be delivered to the same location to increase the size of the first lesion. The resulting PI metric will increase in probability to reflect the greater probability that the lesion size requirements have been satisfied if the electrode delivers the second PFA application to the same location. However, if between delivery of the first PFA application and the second PFA application the catheter and corresponding electrode changes location relative to the tissue, then delivery of the second PFA application will generate a second lesion in a second location and the PI metric from the first lesion should not be aggregated with the PI metric from the second lesion. The location data may be generated via a number of well-known localization/navigation systems utilized in catheter systems, including impedance-based localization systems, magnetic-based localization systems, and others.

Impedance measurements include inputs related to impedance measured by a catheter electrode. The conductivity (inversely, impedance) of tissue varies with lesion formation and therefore can be utilized to monitor lesion formation. In general, lesion formation modifies tissue impedance (e.g., decreases tissue impedance). Measuring a change in tissue impedance can therefore be useful as an input in assessing lesion formation.

Impedance measurements may be measured in a number of ways utilizing a number of circuit configurations. In general, impedance is measured by providing a current of a known amplitude (typically an alternating current (AC) current) between two electrodes and measuring the voltage response. In some examples, the current is provided between two electrodes located on the catheter electrode (e.g., between electrodes 116 and 114 as shown in **FIG. 1B****),** wherein the voltage measured at each electrode (either measured with respect to one another or with respect to a reference electrode) is utilized to measure the impedance between the respective electrodes. In other examples, an AC current of a known amplitude is provided to only one of the electrodes (e.g., electrode 116 shown in **FIG. 1B****),** with a return path being provided through a reference electrode (e.g., surface patch electrode or reference electrode on a reference catheter), wherein a voltage is measured between the electrode and the reference electrode to determine the impedance associated with or seen by the distal electrode 116. In other embodiments, various other configurations may be utilized to measure the impedance associated with one or more of the electrodes.

In some examples, it is important that the measured impedance is representative of measured tissue impedance. For example, measuring impedance between the distal electrode 116 and the second electrode 114 will not be representative of tissue impedance if neither electrode 114, 116 is located adjacent to the tissue. A number of methods may be utilized to ensure that measured impedances are representative of tissue impedance. For example, thresholds may be utilized to filter impedance measurements not indicative of tissue impedance (i.e., impedances measured when the electrode is in the blood pool). Typically, if the electrode measuring impedance (e.g., electrode 114) is located in the blood pool, then the measured impedance will be significantly lower due to the higher conductivity of blood as compared with tissue. In some embodiments, measured impedances are associated with tissue impedance if the measured impedances are above some threshold associated with blood impedances. In other embodiments, measured impedances are associated with tissue impedance if the impedances are measured while contact force (measured as the contact force between the distal electrode 116 and adjacent tissue) is non-zero. Various other methods of confirming that measured impedance is associated with tissue impedance may be utilized.

In some examples, the formation of lesions utilizing PFA therapy decreases the impedance of tissue and therefore tissue impedance - and in particular - changes to tissue impedance values can be utilized as an input to the PI model 300 to assess lesion formation. In some examples, tissue impedance measurements are taken prior to and following delivery of a PFA therapy, and the change in tissue impedance is utilized as an input to assess lesion formation. Changes to tissue impedance typically require a (tissue) impedance measurement prior to delivery of a PFA application and following delivery of the PFA application. For applications in which a plurality of PFA bursts (each PFA burst including a plurality of PFA pulses) are delivered, tissue impedance measurements may be taken between each of the plurality of bursts.

In some embodiments, the impedance measurements may be utilized for reasons other than assessing changes in tissue impedance. For example, an impedance measurement may be utilized to assess proximity of the electrode (e.g., distal electrode 116) to the tissue and/or contact of the electrode with the tissue. Tissue proximity and/or contact based on impedance measurements may be useful in determining whether the electrode was in contact with the tissue during delivery of the PFA therapy.

Contact force measurements include inputs related to the force generated between the catheter utilized to deliver PFA therapies and adjacent tissue. Contact force may be measured by mechanical sensors (e.g., spring mechanisms), fiber-optic sensors, piezoelectric sensors, etc. For catheters in which the distal electrode (e.g., distal electrode 116) delivers the PFA therapy, contact force measurements related to the force provide an indication of whether the electrode delivering the PFA therapy is in contact with the adjacent tissue. In some embodiments, raw contact force measurements may be utilized by the PI model 300. In other embodiments, some variation of the contact force measurements may be utilized, such as average contact force or percentage of time the measured contact force is greater than or equal to a threshold value.

Additional sensor inputs may also be provided as inputs to the PI model 300. Examples of other sensor measurements include temperature measurements, sensed electrocardiogram signals, ultrasound measurements, etc.

The attributes of delivered PFA applications may also be provided as an input to the PI model 300. In some examples, the physician may select from a plurality of PFA therapies each having unique attributes (e.g., duration, pulse width, pulse amplitude, pulse period, total number of bursts, inter-burst duration, electric field, energy-per-pulse, energy-per-burst, and polarity (e.g., monopolar, bipolar). Attributes of the PFA therapy delivered contributes to anticipated lesions created. In embodiments in which only a single type of PFA therapy is available for delivery, then attributes of delivery PFA therapy may not be required as inputs but would be hardcoded into the PFA model 300.

In addition, user inputs may be provided as an input to the PFA model 300. For example, the user may indicate a desired lesion size (e.g., depth, width, or combination of both) or anatomy to receive the PFA therapy. The PI model 300 may be modified based on the lesion size selected such that the PI metric generated by the PI model 300 reflects the probability that a delivered PFA therapy results in a lesion of a desired size.

The PI model 300 in one example is a probabilistic model that generates based on one or more of the available inputs a probability that a lesion satisfies certain requirements (e.g., size thresholds). A probabilistic model is defined herein as a model that calculates the probability of two possible outcomes. A number of probabilistic models may be utilized. In one example, a logistic regression model is utilized as the PI model 300. For example, the PI model 300 calculates the probability of the lesion size requirements being met or not. The PI model 300 generates this probability based on one or more of the received inputs. In addition, the PI model 300 and resulting PI metric is cumulative. That is, assuming PFA applications are delivered in the same location, the PI metric calculated is cumulative, taking into account each previously delivered PFA application as well as inputs received with respect to the previously delivered PFA applications. In some examples, in addition to the PFA applications needing to be delivered to the same location to be cumulative, the PFA applications need to be delivered within a given time interval in order to be cumulative. In one example, the PI metric 314 generated with respect to a given lesion is designed to monotonically increase (i.e., will either stay the same or increase relative to a previous value).

Various combinations of inputs may be utilized by the PI model 300 to generate the PI metric 314. In one example, the inputs utilized by the PI model 300 include attributes of delivered PFA applications and changes in impedance, specifically tissue impedance, provided by the impedance measurement input 304. With respect to the example shown in **FIG. 1B**, the impedance may be measured between distal electrode 116 and second shaft electrode 114. Assuming distal electrode 116 is in partial contact with the tissue in which the ablation was formed, the measured impedance will be representative of the tissue impedance. The formation of lesions within the tissue modifies the tissue impedance and therefore the change in tissue impedance is representative of lesion formation. Likewise, attributes of the delivered PFA application may include number of bursts delivered as part of a PFA application. In this example, the PI model 300 combines the number of bursts delivered with the measured change in tissue impedance to calculate the PI metric 314. The PI metric is cumulative, so the PI metric may increase - indicating an increased probability that a desired lesion size has been reached - in response to additional PFA applications being delivered to the target tissue. For example, a PI metric may be calculated in response to a first PFA application comprised of five bursts being delivered to the target tissue, along with a measured change in tissue impedance. Assuming the calculated PI metric is not greater than or equal to a desired value, a second PFA application may be delivered and an updated PI metric is calculated that takes into account the delivery of both the first and second PFA applications along with the changes in tissue impedance measured across both the first and second PFA applications. In some examples, rather than compare the calculated PI metric to a selected or desired value, the calculated PI metric may be compared to a selected or desired range of values that may allow for the tuning of depth/width trade-offs in predicted lesion dimensions to align with a physician's workflow across tissue of varying thicknesses.

In another example, in addition to attributes of delivered PFA applications and changes in impedance, the PI model 300 utilizes contact force measurements. In some embodiments, contact force measurements are taken during delivery of PFA applications to assess whether the PFA pulses were delivered to the electrode while the electrode was in contact with the target tissue. However, in embodiments in which contact sensors are not active during delivery of PFA pulses, contact force measurements may be taken in an interval prior to delivery of the PFA pulses. In one example, the contact force measurements may be represented as an average contact force measured during the given interval or as a percentage of time the measured contact force is greater than or equal to a threshold value (indicating good contact between the electrode delivering the PFA therapy and the target tissue). As described above, the PI model 300 combines the plurality of inputs in a cumulative fashion to calculate a PI metric indicative of the probability that a lesion of a desired size has been created.

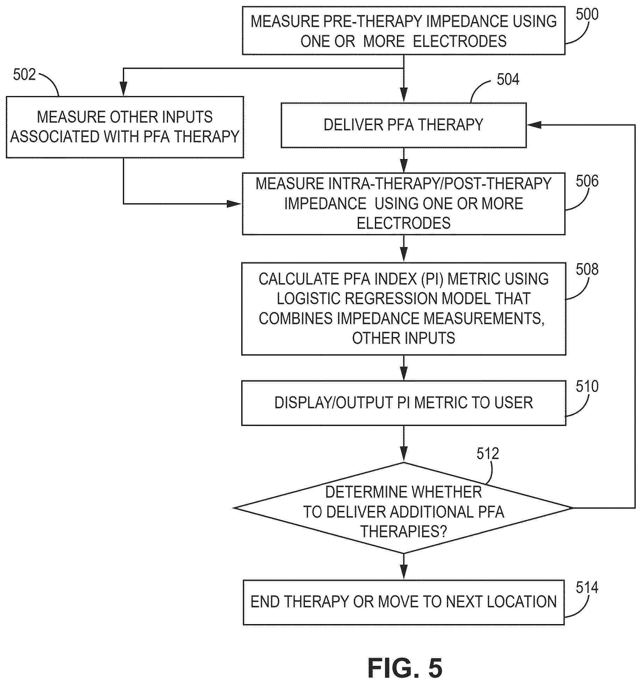

**FIG. 5** is a flowchart illustrating calculation of the PI metric based on received impedance measurements that may be used by the lesion assessment module 36. At step 500, pre-therapy impedance measurements are received from one or more electrodes. As discussed above, impedance measurements may be measured using a variety of methods and configurations, including two-terminal measurements, three-terminal measurements , or other configurations of impedance measurement circuitry. In some examples, the impedance measurements utilized are measured when the electrode is in contact with the target tissue such that the measured impedance represents a tissue impedance.

At step 502, additional inputs are measured. The measuring of additional inputs may be done concurrently with the delivery of PFA therapy at step 504. For example, measuring of additional inputs at step 502 may include measuring of contact force between the electrode delivering the PFA therapy and the adjacent tissue. In some examples, measurement of additional inputs cannot be performed concurrently with the delivery of PFA therapy. In these situations, the additional inputs may be measured prior to delivery of the PFA therapy or subsequent to PFA therapy. For example, contact force may be measured just prior to delivery of the PFA therapy and utilized to infer the contact force between the electrode and the adjacent tissue during delivery of the PFA therapy. The PFA therapy delivered at step 504 may be selected by a physician/user or may be a standard waveform expected to create a lesion of a desired size. In one example, the PFA therapy delivered at step 504 is delivered using at least one of the electrodes utilized to measure the pre-therapy impedance. For example, the distal electrode 116 (shown in **FIG. 1B****)** is utilized to deliver the PFA therapy, then the distal electrode 116 is one of the electrodes utilized to measure the pre-therapy impedance. This is because the impedance measured by distal electrode 116 is utilized to measure the tissue impedance associated with the lesion being formed by the PFA therapy delivered by the distal electrode 116.

At step 506, following delivery of the first PFA therapy, an intra-therapy/post-therapy impedance measurement are received from the one or more electrodes. As described with respect to step 500, impedance measurements may be measured using a variety of methods and configurations. In general, the same method/configuration utilized to measure the impedance at step 500 is utilized at step 504.

At step 508, a PFA index (PI) metric is calculated based on the PFA therapy delivered, the pre-therapy impedance measurements and the intra/post-therapy impedance measurements, and any other additional inputs measured. As described above, the model utilized to generate the PI metric may be a logistic regression model that calculates a probability of two possible outcomes (i.e., lesion satisfies the desired size requirements, lesion does not satisfy the desired size requirements).

At step 510 the PI metric is provided as an output. In some examples, the PI metric is displayed to a user using display 34 (shown in **FIG. 1A****).** For example, a graphical user interface (GUI) may display the location of the lesion with respect to a 3D model of the tissue to be treated. The display may utilize color-coding or shapes to indicate the value of the PI metric to the physician/user.

At step 512, the PI metric is utilized to determine whether to initiate additional PFA therapies. In one example a decision to initiate another PFA therapy at step 504 is automatically initiated in response to the PI metric being less than a threshold value. Likewise, a decision to NOT initiate another PFA therapy and to end the therapy at step 514 is made in response to the PI metric being greater than a threshold. In other embodiments the physician/user manually decides whether to initiate delivery of another PFA therapy based on the displayed PI metric. In some examples, the threshold value may be an adaptive threshold. For example, the adaptive threshold may be modified based on the tissue thickness at a given location or modified based on the expected or actual distance between adjacent lesions. In some examples, a range of threshold values may be utilized in place of a singular threshold value. A range of threshold values allows tuning of depth/width trade-offs in predicted lesion dimensions to align with a physician's workflow across varying tissue thicknesses.

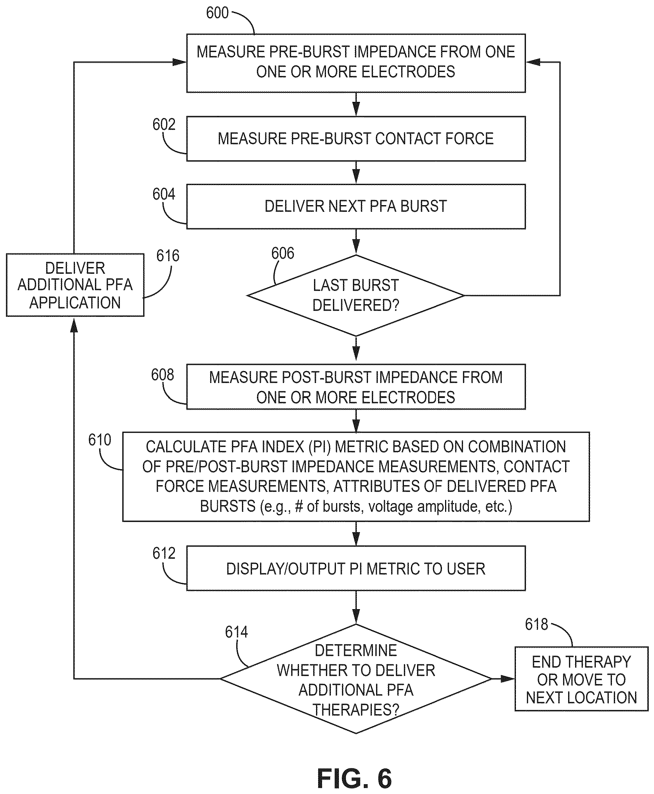

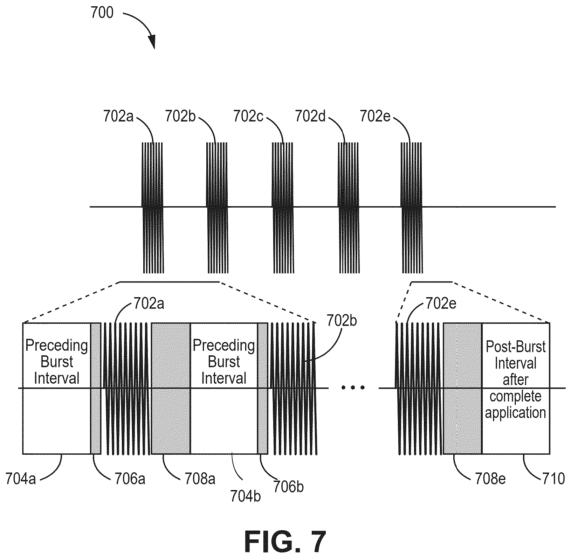

**FIG. 6** is a flowchart illustrating calculation of the PI metric based on impedance measurements, contact force measurements, and attributes of the delivered PFA therapy that may be used by the lesion assessment module 36. The steps described in **FIG. 6** are described with respect to the depiction of therapy illustrated in **FIG. 7****.** In the example shown in **FIG. 7****,** the PFA therapy 700 (single application) includes a plurality of PFA bursts 702a, 702b, 702c, 702d, and 702e. In this example, no measurements are taken during delivery of each PFA bursts 702a-702e. Measurements are taken during pre-burst intervals associated with each PFA bursts 702a-702e and in a post-burst interval 710 following delivery of the last PFA burst 702e. In the example shown in FIG. 7, the pre-burst intervals 704a, 704b (associated with PFA bursts 702a and 702b) are approximately 500ms in length and terminate prior to delivery of the corresponding PFA burst. For example, the first pre-burst interval 704a terminates a set amount of time 706a (e.g., 200ms) prior to delivery of the first PFA burst 702a. Likewise, the second pre-burst interval 704b starts at a set length of time 708a following delivery of the previous PFA burst 702a (e.g., 400 ms). The intervals on either side of each PFA burst ensures that measurements taken are not affected or skewed by the delivery of the adjacent PFA bursts.

At step 600, pre-burst impedance is measured with respect to one or more electrodes. For example, an impedance may be measured between distal electrode 116 and second electrode 114. With respect to the embodiment shown in **FIG. 7****,** the pre-burst impedance is measured during the pre-burst interval 704a associated with PFA burst 702a. In some examples, the pre-burst impedance is representative of tissue impedance. In some examples, the measured impedance is confirmed as representative of tissue impedance based on threshold values (e.g., greater than a minimum value representative of blood pool impedance). In some examples, the pre-burst impedance is confirmed as representative of tissue impedance when the measured contact force is also greater than a threshold value (indicating that the distal electrode is in contact with the target tissue).

At step 602, pre-burst contact force is measured. As described above, this example presumes that contact force cannot be measured during delivery of the PFA bursts as the PFA bursts would interfere with the measurements. In some embodiments, the pre-burst contact force is measured continuously through the pre-burst interval 704a. In one example, the contact force measurements are averaged to generate an average or mean contact force parameter. In another example, the continuously measured contact force is compared to a threshold value and the percentage of time the measured contact force is greater than or equal to the threshold value is utilized as the contact force measurement. For example, if the continuously measured contact force is above the threshold for the duration of the pre-burst interval 704a, the measured contact force would be equal to 100%. If the continuously measured contact force is above the threshold for half of the pre-burst interval 704a, the measured contact force would be equal to 50%.

At step 604, the next PFA burst (e.g., PFA burst 702a) is delivered. At step 606, a determination is made whether the last PFA burst in a given application has been delivered. If not, then the process continues at step 600 with measurement of pre-burst impedance and measurement of pre-burst contact force and delivery of the next PFA burst. In the embodiment shown in **FIG. 7****,** for the five PFA bursts 702a-702e, five pre-burst impedances and pre-burst contact force measurements would be measured. If the last PFA burst in a given application has been delivered (e.g., PFA burst 702e), then at step 608 post-burst impedances are measured corresponding with post-burst interval 710 occurring a set length of time 708e after the end of the last PFA burst 702e. The post-burst impedance measurement utilizes the same techniques described with respect to step 600. In this example, impedance measurements are once again representative of tissue impedances. For measurements such as tissue impedance that are measuring an attribute of the formed lesion a post-burst measurement is useful in assessing lesion formation. For measurements such as contact force, which are related to the position of the electrode relative to the tissue during delivery of the PFA therapy, post-burst measurements are not required.

At step 610, the PI metric is calculated based on a combination of pre-burst impedance measurements, the contact force measurements, the post-burst impedance measurements, and the attributes of the delivered PFA therapy (e.g., number of bursts). In some examples, the PI metric is calculated after delivery of a first PFA application (e.g., after all five bursts 702a-702e have been delivered). In other examples, the PI metric is calculated each time new data is made available (e.g., each time pre-burst impedance measurements and contact force measurements are made). In this example, a PFA application consists of a predetermined number of PFA bursts 702a-702e. That is, while a PI metric may be continually updated based on data made available during pre-burst intervals 704a-704e, the PI metric in this example is not utilized to make determinations of whether additional PFA applications are required until after the last PFA burst (e.g., PFA burst 702e) has been delivered and an updated PI metric is calculated based on data collected during the post-burst interval 710.

In one example, the pre-burst impedance measurements and post-burst impedance measurements are utilized to calculate a change in impedance (e.g., difference between a maximum impedance value and a minimum impedance value). In one example the measured impedances are representative of measured tissue impedance. If the PI metric is being calculated each time new data is made available (e.g., each time a measurement is taken during a pre-burst interval 704a, 704b, etc.), the change in impedance is calculated based on the minimum impedance and maximum impedance measured during each available pre-burst interval. If the PI metric is being calculated only at the end of delivery of all bursts, the change in impedance is calculated based on the minimum impedance and maximum impedance measured during each of the pre-burst intervals 704a-704e and the post-burst interval 710. As described above, in some examples it is important that the impedance measurements are representative of tissue impedance.

In one example, the contact force measurement is represented as a percentage of time the measured contact force is greater than or equal to a threshold value. In other embodiments, the contact force measurement is represented as a mean or average contact force. In some embodiments, the contact force measurement is cumulative of measurements taken during each of the plurality of pre-burst intervals 704a-704e taken prior to delivery of each PFA burst 702a-702e, respectively. In other embodiments, the contact force measurement measured during each pre-burst interval (e.g., pre-burst interval 704a) is utilized to calculate a PI metric associated with the subsequent delivery of the PFA burst (e.g., PFA burst 702a).

In addition, the PI metric is calculated based on attributes of the PFA therapy delivered. In one example, the attribute of the PFA therapy utilized is the number of bursts delivered. For each PFA burst delivered the number of bursts delivered is incremented.

At step 612 the PI metric is displayed/output. In some examples, the PI metric is displayed to a user using display 34 (shown in **FIG. 1A**). For example, a graphical user interface (GUI) may display the location of the lesion with respect to a 3D model of the tissue to be treated. The display may utilize color-coding or shapes to indicate the value of the PI metric to the physician/user.

At step 614, the PI metric is utilized to determine whether to initiate additional PFA therapies. In some examples, the PI metric is compared to a threshold value. If the PI metric is greater than the threshold value then no additional treatment is required and the process ends at step 616 with the physician/user moving the catheter to the next location to be treated. If the PI metric is less than the threshold value then an additional PFA therapy is applied and the process repeats. In some examples, the decision of whether to deliver another PFA therapy is made automatically if the PI metric is less than the threshold. In some examples, the physician/user determines based on the received feedback whether an additional PFA therapy should be delivered.

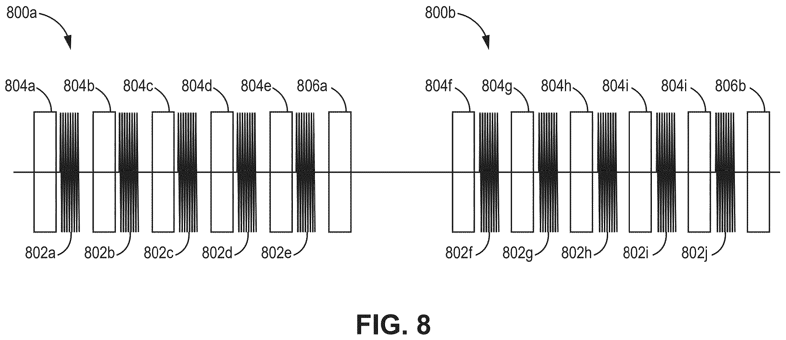

In the example illustrated in **FIG. 7****,** the PFA therapy delivered was comprised of five PFA bursts 702a-702e. In the example illustrated in **FIG. 8****,** the PFA therapy is comprised of a first PFA application 800a and a second PFA application 800b. The first PFA application 800a is comprised of a five bursts 802a-802e and the second PFA application 800b is a comprised of five bursts 802f-802j. In this example, the first PFA application 800a was provided to a tissue target and the PI metric following delivery of the first PFA application 800a was less than a threshold value. As a result, the second PFA application 800b was provided to the tissue target. In one example, measurements are taken during each of the pre-burst intervals 804a-804j and during the first post-burst interval 806a (following the first PFA application 800a) and the second post-burst interval 806b (following the second PFA application 800b).

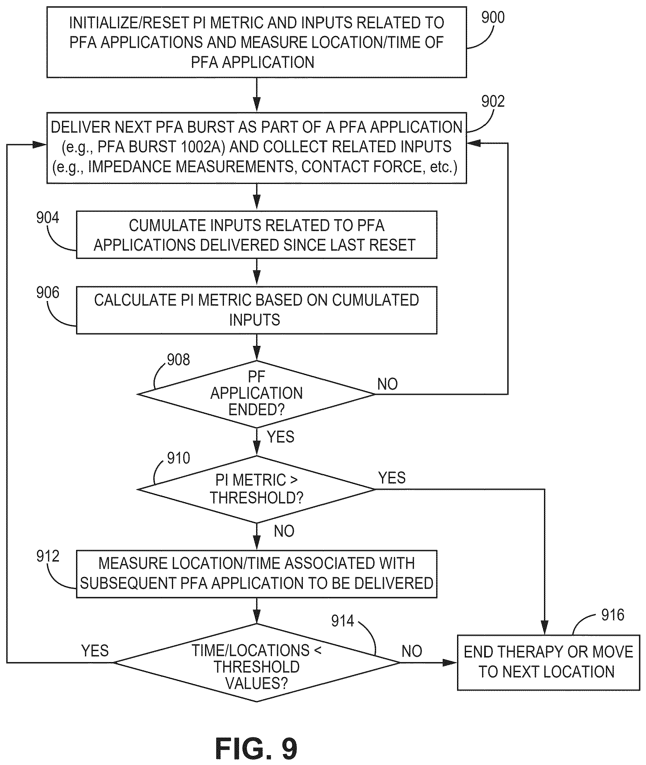

In one example, the measurements across the first and second PFA applications 800a, 800b are cumulative. For example, the contact force measurement utilized to calculate the final PI metric (following the post-burst interval 806b) would include the percentage of time the measured contact force (measured during each of the plurality of pre-burst intervals 804a-804e and 804f-804j) was greater than a threshold value. In other embodiments, the contact force may be a mean or average of the measured contact force across each of the plurality of pre-burst intervals 804a-804j. Likewise, the change in measured impedance may be cumulative across the plurality of pre-burst intervals 804a-804j and the plurality of post-burst intervals 806a and 806b. As discussed in more detail with respect to **FIG. 9****,** rules for determining whether measurements taken with respect to a first PFA application 800a and a second PFA application 800b (or subsequent PFA application, not shown) are cumulative include whether the first and second PFA applications were delivered to approximately the same location (e.g., difference in location less than some threshold) and delivered within a given interval of time (e.g., less than some threshold of time).

For measurements like the change in impedance - based on the minimum measured impedance and maximum measured impedance - the measurement cannot decrease in value, only grow. However, for other measurements such as contact force the cumulative measurement may decrease in value. Under some circumstances the PI metric calculated based on these inputs may decrease in value despite additional PFA therapies being delivered. To prevent this counterintuitive output from being provided to a user, in some examples monotonicity rules are enforced on the PI metric displayed or output to users. For example, a monotonicity rule may filter a raw PI metric to prevent decreases in the output or displayed PI metric.

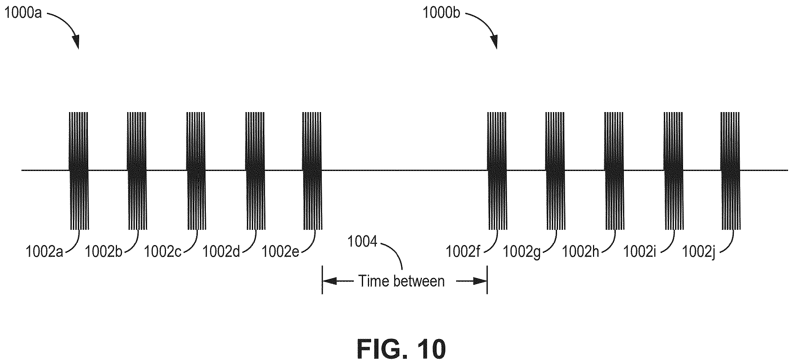

**FIG. 9** is a flowchart illustrating the aggregation of inputs measured over two or more PFA applications based on timing/location thresholds. The steps described in **FIG. 9** are described with respect to the depiction of therapy illustrated in **FIG. 10****.** In the example illustrated in **FIG. 10****,** the PFA therapy is comprised of a first PFA application 1000a and a second PFA application 1000b. The first PFA application 1000a is comprised of a five bursts 1002a-1002e and the second PFA application 1000b is a comprised of five bursts 1002f-1002j. In this example, the first PFA application 1000a was provided to a tissue target and the PI metric following delivery of the first PFA application 1000a was less than a threshold value. As a result, the second PFA application 1000b was provided to the tissue target. **FIG. 10** illustrates the conditions required to be met to determine whether the PI metric should be cumulative across both PFA applications 1000a, 1000b. In particular, if the location of the electrode delivering the first and second PFA applications 1000a, 1000b has changed by more than a threshold distance then the PI metric should be reset with respect to delivery of the second PFA application 1000b. Likewise, if the interval of time 1004 between delivery of the first PFA therapy 1000a and the second PFA therapy 1000b is greater than a threshold amount then the PI metric is reset with respect to delivery of the second PFA application 1000b. In the example shown in **FIG. 10****,** the threshold time interval 1004 is measured between the end of the last PFA burst 1002e delivered with respect to the first PFA application 1000a and the beginning of the first PFA burst 1002f delivered with respect to the second PFA application 1002f.

**FIG. 9** is a flowchart illustrating aggregation of lesion assessment inputs over multiple pulsed field ablation (PFA) applications based on spatial and temporal criteria. At step 900, the PFA index (PI) metric and all corresponding inputs (e.g., impedance, contact force, and other lesion assessment parameters) are initialized or reset prior to delivery of a PFA application (e.g., first PFA application 1000a shown in **FIG. 10****).** In addition, the location of the device (or more particularly, the location of the electrode or electrodes delivering therapy) are measured along with the time associated with delivery of the next PFA application (e.g., PFA application 1000a). In one example, the location associated with the first PFA application 1000a is calculated as the median location measured during the duration delivery of all bursts 1002a-1002e comprising the first PFA application 1000a. In other examples, the location may be measured prior to delivery of the first PFA application 1000a, during the delivery of the first PFA application 1000a, or following delivery of the first PFA application.

At step 902, a PFA burst (e.g., 1002a) included as part of a PFA application (e.g., PFA application 1000a) is delivered and related inputs are collected (e.g., impedance measurements, contact force, etc.). Collected inputs may include data collected pre-burst or post-burst.

At step 904, inputs related to PFA applications delivered since the last reset are cumulated. This may include inputs collected pre-burst with respect to a number of PFA bursts (e.g., PFA bursts 1002-1002e associated with first PFA application 1000a), inputs collected post-burst with respect to a given PFA application (e.g., PFA application 1000a), or may include inputs collected with respect to previous PFA applications. For example (assuming the PI metric and other inputs have not been initialized/reset at step 900), inputs collected with respect to separate PFA applications (e.g., collected during first PFA application 1000a and second PFA application 1000b) may be cumulated so long as location/time conditions are met as described below.

At step 906, the PI metric is calculated based on the inputs collected and cumulated at step 904 as well as any additional inputs related to the calculation of the PI metric as discussed above. In this example, the PI metric is updated each time new inputs are available, which includes updating the PI metric during delivery of each of the plurality of individual bursts (e.g., bursts 1002a-1002e) using inputs collected pre-burst as well as updating the PI metric following delivery of a PFA application (e.g., PFA application 1000a) using inputs collected both pre-burst and post-burst.

At step 908 a determination is made whether the PFA application has ended. For example, with respect to the first PFA application shown in FIG. 10, the PFA application 1000a is ended following the delivery of the last burst 1002e. If the PFA application has not ended, then the process returned to step 902 and the next PFA burst included as part of a PFA application is delivered. If the PFA application has ended, then at step 910 the PI metric calculated at step 906 is compared to a threshold value or range of values to determine whether additional treatment is required. In some examples, particularly if PI metric is compared to a range of values, a physician or operator may make the final determination of whether the delivered PFA application is sufficient or whether additional PFA applications are required. If at step 910 the PI metric is greater than the threshold (or otherwise satisfactory), then at step 916 the therapy at that particular location is ended. If the therapy is ended at that location, then at step 900 the PI metric and related inputs are initialized or reset and the process continues. If at step 910 the PI metric is less than the threshold (or otherwise deemed unsatisfactory), then at step 912 a decision to deliver a subsequent or second PFA application (e.g., PFA application 1000b) is made and at step 912 one or both of location and time of the subsequent or second PFA application is measured. As described above, the location may include the location of the device delivering the second or subsequent PFA application or more particularly the location of the electrode or electrodes that will be delivering the second or subsequent PFA application. In one example, the median location measured across all bursts of the first PFA application 1000a is compared to the location of the electrodes measured prior to delivery of the second PFA application 1000b to determine displacement of the electrodes between respective first and second PFA applications 1000a, 1000b.

At step 914, the location and/or time information collected at step 912 is compared to threshold values to determine whether to cumulate PI metrics and other inputs. In particular, if the location of the device or the electrodes delivering the therapy have moved significantly between delivery of a previous PFA application (e.g., PFA application 1000a) and delivery of a subsequent PFA application (e.g., PFA application 1000b) then the change in location will be greater than a threshold value and the PI metric and other inputs cannot be cumulated. Likewise, if the difference in time between the previous PFA application (e.g., PFA application 1000a) and the subsequent PFA application (e.g., PFA application 1000b) is greater than a threshold value then the PI metric and other inputs cannot be cumulated. In some examples, only location information is utilized to determine whether PI metrics and other inputs can be cumulated with respect to successive PFA applications. In other examples, both location and time information are utilized to determine whether PI metrics and other inputs can be cumulated.

If it is determined that the PI metric and other inputs can be cumulated at step 914 because the change in location is less than a threshold value and/or the time between PFA applications is less than a threshold value, then the method continues at step 902 with delivery of the next PFA burst associated with the next PFA application and subsequent cumulating of collected inputs and PI metrics. With respect to the example shown in FIG. 10, assuming the change in location of the electrodes configured to deliver the PFA applications 1000a, 1000b is less than a threshold value and the time elapsed between delivery of the first PFA application 1000a and the second PFA application 1000b (e.g., measured between the end of the last burst 1002e of the first PFA application 1000a and the start of the first burst 1002f associated with the second PFA application 1000b as indicated by time duration 1004) then the PI metric calculated with respect to the first PFA application 1000a is not reset but instead allowed to monotonically increase as the second PFA application 1000b is delivered. Likewise, the inputs utilized to calculate the PI metric are also cumulated with respect to the first PFA application 1000a and the second PFA application 1000b.

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

Clause 1. A computer-implemented method of assessing lesion formation associated with pulsed field ablation (PFA) therapy delivered to a selected location by a PFA catheter, the method comprising: measuring pre-therapy impedance using one or more electrodes located on the catheter; measuring intra-therapy/post-therapy impedance using the one or more electrodes located on the catheter; and calculating a PFA index (PI) metric based on a model that combines the measured pre-therapy impedance, the measured intra-therapy/post-therapy impedance, and attributes of a delivered PFA therapy, wherein the PI metric is representative of a likelihood that the lesion has reached a selected size.

Clause 2. The method of clause 1, wherein the measured pre-therapy impedance and measured intra-therapy/post-therapy impedance is representative of tissue impedance associated with tissue receiving the delivered PFA therapy.

Clause 3. The method of clause 1 or 2, wherein the model utilizes a change in tissue impedance calculated on a minimum tissue impedance measured and a maximum tissue impedance measured.

Clause 4. The method of any of clauses 1-3, further including: measuring one or more other inputs associated with the delivered PFA therapy, wherein the model further combines the one or more other inputs in generating the PI metric.

Clause 5. The method of clause 4, wherein the one or more other inputs includes contact force measured between the catheter and adjacent tissue.

Clause 6. The method of clause 5, wherein contact force is measured as a percentage of time the measured contact force is greater than or equal to a threshold value.

Clause 7. The method of any of clauses 1-6, wherein the PI metric calculated by the model is filtered to only permit increases in value of the PI metric.

Clause 8. The method of any of clauses 1-7, wherein the delivered PFA therapy includes a first PFA application comprised of a first plurality of PFA bursts, wherein the pre-therapy impedance and intra-therapy impedances are measured during a pre-burst interval preceding each of the first plurality of PFA bursts, wherein a post-burst impedance is measured after a final PFA burst delivered as part of the first PFA application.

Clause 9. The method of clause 8, wherein attributes of the first PFA application delivered utilized by the model includes a number of PFA bursts delivered.

Clause 10. The method of clause 8 or 9, further including: measuring one or more additional inputs during the pre-burst intervals preceding each of the first plurality of PFA bursts, wherein the one or more additional inputs includes contact force.

Clause 11. The method of any of clauses 8-10, further includes: delivering as second PFA application to the selected location if the PI metric is less than a threshold value, wherein the second PFA application includes a second plurality of PFA bursts.

Clause 12. The method of any of clauses 8-11, further including: measuring first contact force associated with the catheter during delivery of the first PFA application delivered; measuring second contact force associated with the catheter during delivery of the second PFA application delivered, wherein calculating the PI metric following delivery of the second PFA application utilizes a cumulative contact force based on the first contact force and the second contact force.

Clause 13. A system for assessing lesion formation associated with pulsed field ablation (PFA) therapy delivered to a selected location by a PFA catheter, the system comprising: a lesion assessment module running on a computer system, the lesion assessment module configured to: receive pre-therapy impedances measured using one or more electrodes located on the catheter; receive intra-therapy/post-therapy impedances mesaured using the one or more electrodes located on the catheter; and calculate a PFA index (PI) metric based on a model that combines the measured pre-therapy impedance, the measured intra-therapy/post-therapy impedance, and attributes of the delivered PFA therapy, wherein the PI metric is representative of a likelihood that the lesion has reached a given size.

Clause 14. The system of clause 13, wherein the measured pre-therapy impedance and measured intra-therapy/post-therapy impedance is representative of tissue impedance associated with tissue receiving the delivered PFA therapy.

Clause 15. The system of clause 13 or 14, wherein the model utilizes a change in tissue impedance calculated on a minimum tissue impedance measured and a maximum tissue impedance measured.

Clause 16. The system of any of clauses 13-15, wherein the lesion assessment module is further configured to: receive one or more other inputs measured with respect to the delivered PFA therapy, wherein the model further combines the one or more other inputs in generating the PI metric.

Clause 17. The system of clause 16, wherein the one or more other inputs measured includes contact force measured between the catheter and adjacent tissue.

Clause 18. The system of clause 17, wherein contact force is measured as a percentage of time the measured contact force is greater than or equal to a threshold value.

Clause 19. The system of any of clauses 13-18, wherein the PI metric calculated by the model is filtered to only permit increases in value of the PI metric.

Clause 20. The system of any of clauses 13-19, wherein the delivered PFA therapy includes a first PFA application comprised of a first plurality of PFA bursts, wherein the pre-therapy impedance and intra-therapy impedances are measured during a pre-burst interval preceding each of the first plurality of PFA bursts, wherein a post-burst impedance is measured after a final PFA burst delivered as part of the first PFA application.

Clause 21. The system of clause 20, wherein attributes of the first PFA application delivered utilized by the model includes a number of PFA bursts delivered.

Clause 22. The system of clause 20 or 21, wherein the lesion assessment module is further configured to: receive one or more additional inputs measured during the pre-burst intervals preceding each of the first plurality of PFA bursts, wherein the one or more additional inputs includes contact force.

Clause 23. The method of any of clauses 20-22, wherein the lesion assessment module is further configured to: receive a first contact force associated with the catheter during delivery of a first PFA application delivered; receive a second contact force associated with the catheter during delivery of a second PFA application, wherein calculating the PI metric following delivery of the second PFA application utilizes a cumulative contact force based on the first contact force and the second contact force.

Clause 24. A computer implemented method of assessing lesion formation associated with pulsed field ablation (PFA) therapy delivered to a selected location by an electrode located on a catheter, the method comprising: measuring tissue impedance both prior to and after delivery of a first PFA application to determine a change in tissue impedance; measuring contact force between the catheter and adjacent tissue prior to delivery of the first PFA application; and calculating a PFA index (PI) metric based on a model that combines the change in tissue impedance, the contact force, and one or more attributes of the first PFA application, wherein the PI metric is representative of a likelihood that the lesion has reached a selected size.

Clause 25. The computer implemented method of clause 24, further comprising delivering a first PFA application to the selected location.

Clause 26. The computer implemented method of clause 25, wherein attributes of the first PFA application delivered utilized by the model includes a number of PFA bursts delivered, and wherein the system is configured to deliver a second PFA application to the selected location if the PI metric is less than a threshold value, wherein the second PFA application includes a second plurality of PFA bursts.

Clause 27. The method of clause 26, wherein the model is a logistic regression model that maps the measured change in tissue impedance, the measured contact force, and the one or more attributes of the first PFA application to a probability the lesion has reached the selected size.

Clause 28. The method of clause 26 or 27, further including: receiving an input from a user regarding the selected lesion size; and modifying the model to generate a PI metric representative of a likelihood that the lesion has reached the selected size.

Clause 29. The method of clause 26, 27, or 28, wherein the first PFA application is comprised of a plurality of bursts.

Clause 30. The method of clause 29, wherein the tissue impedance is measured before each burst and after a final burst of the first PFA application and the contact force is measured before each burst.

Clause 31. The method of any of clauses 26-30, wherein change in tissue impedance is calculated by identifying a minimum tissue impedance and a maximum tissue impedance measured and subtracting the minimum tissue impedance from the maximum tissue impedance.

Clause 32. The method of any of clauses 26-30, wherein the contact force is calculated as a percentage of time across all measurements that the contact force is above a threshold.

Clause 33. The method of any of clauses 26-32, wherein a second PFA application is delivered if the PI metric is less than a threshold value.

Clause 34. A method of assessing lesion formation associated with pulsed field ablation (PFA) therapy delivered to a selected location by a PFA catheter, the method comprising: measuring pre-therapy impedance using one or more electrodes located on the catheter; delivering a first PFA application; measuring intra-therapy/post-therapy impedance using the one or more electrodes located on the catheter; and calculating a PFA index (PI) metric based on a model that combines the measured pre-therapy impedance, the measured intra-therapy/post-therapy impedance, and attributes of the first PFA application delivered, wherein the PI metric is representative of a likelihood that the lesion has reached a selected size.

Clause 35. A system for assessing lesion formation associated with pulsed field ablation (PFA) therapy delivered to a selected location by an electrode located on a catheter, the system comprising a lesion assessment module running on a computer system, the lesion assessment module configured to: measure tissue impedance both prior to and after delivery of a first PFA application to determine a change in tissue impedance; measure contact force between the catheter and adjacent tissue prior to delivery of the first PFA application; and calculate a PFA index (PI) metric based on a model that combines the change in tissue impedance, the contact force, and one or more attributes of the first PFA application, wherein the PI metric is representative of a likelihood that the lesion has reached a selected size.

Clause 36. A method of assessing lesion formation associated with pulsed field ablation (PFA) therapy delivered to a selected location by an electrode located on a catheter, the method comprising: delivering a first PFA application to the selected location, the first PFA application defined by one or more attributes; measuring tissue impedance both prior to and after delivery of the first PFA application to determine a change in tissue impedance; measuring contact force between the catheter and adjacent tissue prior to delivery of the first PFA application; and calculating a PFA index (PI) metric based on a model that combines the change in tissue impedance, the contact force, and the one or more attributes of the first PFA application, wherein the PI metric is representative of a likelihood that the lesion has reached a selected size.

## Claims

1. A computer-implemented method of assessing lesion formation associated with pulsed field ablation (PFA) therapy delivered to a selected location by a PFA catheter (12), the method comprising:
measuring pre-therapy impedance using one or more electrodes (112, 114, 116) located on the catheter (12) (step 500);
measuring intra-therapy/post-therapy impedance using the one or more electrodes (112, 114, 116) located on the catheter (12) (step 506); and
calculating a PFA index (PI) metric (314) based on a model (300) that combines the measured pre-therapy impedance, the measured intra-therapy/post-therapy impedance, and attributes of a delivered PFA therapy (step 508), wherein the PI metric (314) is representative of a likelihood that the lesion has reached a selected size.

2. The computer-implemented method of claim 1, wherein the model (300) utilizes a change in tissue impedance calculated based on a minimum tissue impedance measured and a maximum tissue impedance measured.

3. The computer-implemented method of claims 1 or 2, further including:
measuring one or more other inputs associated with the delivered PFA therapy (step 502), wherein the model (300) further combines the one or more other inputs in generating the PI metric (314).

4. The computer-implemented method of claim 3, wherein the one or more other inputs includes contact force (306) measured between the catheter and adjacent tissue (step 602).

5. The computer-implemented method of claim 4, wherein contact force (306) is measured as a percentage of time the measured contact force is greater than or equal to a threshold value.

6. The computer-implemented method of any preceding claim, wherein the delivered PFA therapy includes a first PFA application (700, 800a, 1000a) comprised of a first plurality of PFA bursts (702a-702e, 802a-802e, and 1002a-1002e), wherein the pre-therapy impedance and intra-therapy impedances are measured during a pre-burst interval (704a-704e, 804a-804e) preceding each of the first plurality of PFA bursts (702a-702e, 802a-802e, and 1002a-1002e), wherein a post-burst impedance is measured after a final PFA burst (702e, 802e, 1002e) delivered as part of the first PFA application (700, 800a, 1000a), wherein attributes of the first PFA application (700, 800a, 1000a) delivered utilized by the model includes a number of PFA bursts delivered.

7. The computer-implemented method of claim 6, further including:
measuring one or more additional inputs during the pre-burst intervals (704a-704e, 804a-804e) preceding each of the first plurality of PFA bursts (702a-702e, 802a-802e, and 1002a-1002e), wherein the one or more additional inputs includes contact force.

8. The computer-implemented method of claims 6 or 7, further including:
delivering a second PFA application (800b, 1000b) to the selected location if the PI metric (314) is less than a threshold value (step 910), wherein the second PFA application (800b, 1000b) includes a second plurality of PFA bursts (802f-802j, 1002f-1002j).

9. The computer-implemented method of claims 6, 7, or 8, further including:
measuring first contact force associated with the catheter during delivery of the first PFA application (800a, 1000a) delivered;
measuring second contact force associated with the catheter during delivery of the second PFA application (800b, 1000b) delivered, wherein calculating the PI metric (314) following delivery of the second PFA application (800b, 1000b) utilizes a cumulative contact force based on the first contact force and the second contact force (step 904).

10. A system for assessing lesion formation associated with pulsed field ablation (PFA) therapy delivered to a selected location by a PFA catheter (12), the system (10) comprising:
a lesion assessment module (36) running on a computer system (18), the lesion assessment module (36) configured to:
receive pre-therapy impedances measured using one or more electrodes (112, 114, 116) located on the catheter (step 500);
receive intra-therapy/post-therapy impedances measured using the one or more electrodes (112, 114, 116) located on the catheter (step 506); and
calculate a PFA index (PI) metric based on a model (300) that combines the measured pre-therapy impedance, the measured intra-therapy/post-therapy impedance, and attributes of the delivered PFA therapy (step 508), wherein the PI metric (314) is representative of a likelihood that the lesion has reached a given size.

11. The system of claim 10, wherein the lesion assessment module (36) is further configured to:
receive one or more other inputs measured with respect to the delivered PFA therapy (step 502), wherein the model (300) further combines the one or more other inputs in generating the PI metric (314).

12. The system of claim 11, wherein the one or more other inputs measured includes contact force measured between the catheter and adjacent tissue (step 602), wherein contact force is measured as a percentage of time the measured contact force is greater than or equal to a threshold value.

13. The system of any of claims 10 to 12, wherein the delivered PFA therapy (700) includes a first PFA application (700, 800a, 1000a) comprised of a first plurality of PFA bursts (702a-702e, 802a-802e, and 1002a-1002e), wherein the pre-therapy impedance and intra-therapy impedances are measured during a pre-burst interval (704a-704e, 804a-804e) preceding each of the first plurality of PFA bursts (702a-702e, 802a-802e, and 1002a-1002e), wherein a post-burst impedance is measured after a final PFA burst (702e, 802e, 1002e) delivered as part of the first PFA application (700, 800a, 1000a), wherein attributes of the first PFA application (700, 800a, 1000a) delivered utilized by the model includes a number of PFA bursts delivered.

14. The system of claim 13, wherein the lesion assessment module (36) is further configured to:
receive one or more additional inputs measured during the pre-burst intervals (704a-704e, 804a-804e) preceding each of the first plurality of PFA bursts (702a-702e, 802a-802e, and 1002a-1002e), wherein the one or more additional inputs includes contact force.

15. The system of claims 13 or 14, wherein the lesion assessment module (36) is further configured to:
receive a first contact force associated with the catheter during delivery of a first PFA application (800a, 1000a) delivered;
receive a second contact force associated with the catheter during delivery of a second PFA application (800b, 1000b), wherein calculating the PI metric (314) following delivery of the second PFA application (800b, 1000b) utilizes a cumulative contact force based on the first contact force and the second contact force.
